# EUROPEAN PATENT SPECIFICATION

(11) **EP 2 692 848 B1**
(45) Date of publication and mention of the grant of the patent: **25.03.2015**
(21) Application number: 11862175.4
(22) Date of filing: 22.12.2011
(51) Int. Cl.: C12M 1/12, A61L 2/20, B05B 7/04, B05B 7/30, A61L 2/18

(54) **DECONTAMINATION SOLUTION SPRAY DEVICE**
SPRAYVORRICHTUNG FÜR EINE DEKONTAMINIERUNGSLÖSUNG
DISPOSITIF DE PULVÉRISATION DE SOLUTION DE DÉCONTAMINATION

(30) Priority: 29.03.2011 JP 2011072853
(43) Date of publication of application: 05.02.2014
(73) Proprietor: Panasonic Healthcare Holdings Co., Ltd., Tokyo 105-8433 (JP)
(72) Inventor: NAKATA Yuji, Chuo-ku, Osaka 540-6207 (JP); KURUSU Koichi, Chuo-ku, Osaka 540-6207 (JP); SATOH Keiji, Chuo-ku, Osaka 540-6207 (JP); IWAMA Akifumi, Chuo-ku, Osaka 540-6207 (JP)
(74) Representative: Grünecker, Kinkeldey, Stockmair & Schwanhäusser
(86) International application number: PCT/JP2011/079761
(87) International publication number: WO 2012/132146

(56) References cited:
- EP-A1- 2 210 618
- EP-A1- 2 286 846
- EP-A1- 2 517 734
- EP-A1- 2 517 735
- JP-A- 2003 339 829
- JP-A- 2006 320 392
- JP-A- 2010 194 301
- JP-A- 2011 030 654
- JP-A- 2011 030 655

## Description

### [Technical Field]

The present invention relates to a decontamination solution spray device.

### [Background Art]

A cell culture apparatus, an isolator, and the like include a decontaminating gas generating device configured to gasify decontamination solution such as hydrogen peroxide solution and generate decontaminating gas such as hydrogen peroxide gas. Various techniques to generate decontaminating gas have been developed (see Document 1, for example).

The technique described in Document 1 is to generate decontaminating gas, by mixing air heated by a heater and decontamination solution pumped up by a pump with a spray, and atomizing using the spray.

### [Citation List]

### [Patent Literature]

EP 2 286 846 A1 relates to a hydrogen peroxide supplying apparatus having an atomization unit, an ultrasound vibrator, and an evaporation unit. EP 2 210 618 Al relates to a bio isolator having a sterilized workroom.

[PTL 1] Japanese Patent Application Laid-Open Publication No. 2003-339829

### [Summary of Invention]

### [Technical Problem]

However, if air supply to a spray is not carried out appropriately, there exists a possibility that the decontamination solution delivered under pressure from a pump is not appropriately atomized, allowing direct injection of the decontamination solution in the form of liquid or dripping thereof.

The present invention has been made in view of such a problem, and an object thereof is to prevent direct injection and dripping of the decontamination solution, even when air supply to a spray is not carried out appropriately.

### [Solution to Problem]

In order to achieve the above object, a decontamination solution spray device according to one aspect of the present invention is defined in claim 1.

### [Advantageous Effects of Invention]

Direct injection and dripping of decontamination solution is prevented even when air supply to a spray is not carried out appropriately.

### [Brief Description of Drawings]

Fig. 1 is a diagram illustrating a configuration of an isolator 10 according to a first embodiment of the present invention.
Fig. 2 is a diagram illustrating functional blocks implemented in a microcomputer 71.
Fig. 3 is a diagram illustrating a configuration of an isolator 10 according to a second embodiment of the present invention.
Fig. 4 is a diagram illustrating a configuration of an isolator 10 according to a third embodiment of the present invention.

### [Description of Embodiments]

At least the following details will become apparent from descriptions of the present specification and of the accompanying drawings.

Note that, in the present specification, killing microorganisms, bacteria, and the like for approaching an asepsis state is referred to as decontamination, and the meaning of the term includes so-called decontamination, decolonization, disinfection, and the like.

### == First Embodiment ==

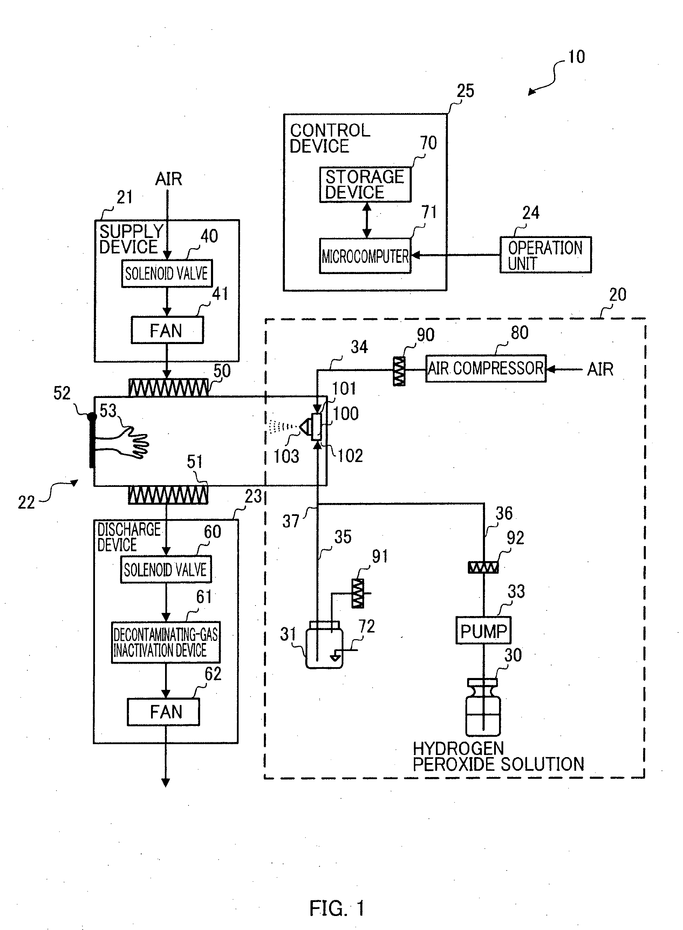

Fig. 1 is a diagram illustrating a configuration of an isolator 10 according to a first embodiment of the present invention. The isolator 10 is a device configured to conduct work on cells under a decontaminated environment, and includes a decontamination solution spray device 20, a supply device 21, a working chamber 22, a discharge device 23, an operation unit 24, and a control device 25.

### <Decontamination Solution Spray Apparatus>

The decontamination solution spray device 20 is a device unit configured to spray decontamination solution to the interior of the working chamber 22, and includes an atomizer 100, a tank (reservoir portion) 30, a bottle 31, a pump 33, a first pipe 34, a second pipe 35, a thirdpipe 36, an air compressor 80, air filters 90, 91 and a filter 92.

Further, the bottle 31 is provided with a water-level sensor 72. The pump 33 and the air compressor 80 are controlled by the control device 25.

The air compressor 80 takes in air from the exterior and delivers under pressure air to the first pipe 34 when receiving from the control device 25 an instruction to start operation.

The first pipe 34 has one end connected to the air compressor 80, and the other end connected to a first port 101 of the atomizer 100. In this way, air delivered under pressure from the air compressor 80 is supplied to the first port 101 of the atomizer 100 through the first pipe 34.

Note that the air filter 90 is provided on the path of the first pipe 34, and impurities such as dust and moisture in the air sent out from the air compressor 80 are removed by the air filter 90.

The atomizer 100 includes the first port 101, a second port 102, and a nozzle 103. Each of the first port 101 and the second port 102 is in fluid communication with the nozzle 103 through a flow path formed in the interior of the atomizer 100.

Further, the nozzle 103 is formed to have a diameter smaller than the diameter of the first port 101. Thus, the air having flown in from the first port 101 to the atomizer 100 is accelerated on the flow path in the interior of the atomizer 100, and is injected from the nozzle 103.

The second port 102 of the atomizer 100 is connected to the second pipe 35.

The second pipe 35 is provided at a location lower than the second port 102 (at a position with lower potential energy), and one end thereof is connected to the second port 102 of the atomizer 100 and the other end thereof is open.

In an embodiment of the present invention, the other end of the second pipe 35 is open in the space inside the bottle 31 provided vertically below the other end of the second pipe 35. The space inside the bottle 31 is open to the atmosphere through an air filter 91.

The air, supplied from the air compressor 80 to the first port 101 of the atomizer 100, increases the flow rate thereof in the interior of the atomizer 100, and is sprayed from the nozzle 103, as described above. When the flow rate of the air increases in the interior of the atomizer 100, the pressure in the flow path leading from the first port 101 to the nozzle 103 becomes lower than atmospheric pressure (negative pressure). As a result, the pressure at the second port 102 in fluid communication with this flow path also becomes lower than atmospheric pressure, thereby causing the atmosphere to flow into the second port 102 through the second pipe 35 connected to the second port 102. The atmosphere having flown into the second port 102 through the second pipe 35 joins the air having flown in from the first port 101, in the flow path of the interior of the atomizer 100, and is sprayed from the nozzle 103.

Whereas, the tank 30 stores, for example, hydrogen peroxide solution (solution in which hydrogen peroxide (H₂O₂) is dissolved) as decontamination solution.

The pump 33 is operated under control of the control device 25, and pumps up the hydrogen peroxide solution from the tank 30 and sends it out to the third pipe 36.

The filter 92 is provided on the path of the third pipe 36. The filter 92 removes impurities such as dust in the hydrogen peroxide solution sent out from the pump 33.

The third pipe 36 has one end connected to the pump 33, and the other end joined to the second pipe 35 at a junction 37 on the path of the second pipe 35.

Since the second pipe 35 is provided at a location lower than the second port 102 of the atomizer 100, as described above, the junction 37 is located below the second port 102. Further, the other end of the second pipe 35 is also in a position lower than the second port 102.

Thus, the hydrogen peroxide solution, sent out by the pump 33 through the third pipe 36 to the junction 37, cannot rise up to the second port 102 of the atomizer 100 by only the force of the pump 33.

However, as described above, since the pressure at the second port 102 of the atomizer 100 is lower than atmospheric pressure (negative pressure), the hydrogen peroxide solution sent out to the junction 37 by the pump 33 rises toward the second port 102 of the atomizer 100 by this pressure difference.

The hydrogen peroxide solution delivered up to the second port 102 of the atomizer 100, merges with the air having flown into the atomizer 100 from the first port 101, and is injected from the nozzle 103 in the form of a mist gas.

As such, decontamination of the interior of the working chamber 22 can be performed effectively. For example, hydrogen peroxide solution gas in the atomized state can be generated effectively and sprayed into the working chamber 22 without the use of a heater or an ultrasonic vaporizer when atomizing the hydrogen peroxide solution.

Further, in the decontamination solution spray device 20 according to an embodiment of the present invention, even if the pump 33 keeps operating although air supply to the atomizer 100 stops due to some failure, for example, the hydrogen peroxide solution sent out from the pump 33 can be discharged from the other end of the second pipe 35, without the hydrogen peroxide solution being raised up to the second port 102 of the atomizer 100. As a result, the hydrogen peroxide solution can be reliably prevented from being directly injected in the form of liquid to the interior of the working chamber 22. Further, dripping of the solution out of the atomizer 100 can be prevented.

Thus, a worker can safely conduct work such as cell culture in the working chamber 22 without the hydrogen peroxide solution being directly injected in the form of liquid to a sample in the interior of the working chamber 22.

Further, as illustrated in Fig. 1, in the decontamination solution spray device 20 according to an embodiment of the present invention, the bottle 31 is provided vertically below the other end of the second pipe 35, and the hydrogen peroxide solution having run down the second pipe 35 is stored in the bottle 31. Thus, the hydrogen peroxide solution can be collected safely.

Further, the decontamination solution spray device 20 according to an embodiment of the present invention includes the water-level sensor 72 provided for the bottle 31. When a predetermined amount of the hydrogen peroxide solution has been stored in the bottle 31, the water-level sensor 72 detects that effect, and outputs a signal indicative of the detection to the control device 25.

Then, the control device 25 stops the pump 33 when receiving, from the water-level sensor 72, a signal indicating that the predetermined amount of the hydrogen peroxide solution has been stored in the bottle 31. As a result, supply of the hydrogen peroxide solution can be stopped before the hydrogen peroxide solution overflows out of the bottle 31, thereby being able to improve safety of the isolator 10.

Further, by stopping the pump 33 after the predetermined amount of the hydrogen peroxide solution is stored in the bottle 31, excessive stops of the pump 33 can be prevented even when the capability of the air compressor 80 is reduced due to temporary changes in environmental conditions, such as a temporary change in external power supply voltage, thereby causing the hydrogen peroxide solution to run down due to insufficient air and the like. As a result, operation efficiency can be maintained with safety of the isolator 10 being secured.

### <Supply Device>

The supply device 21 is a device configured to supply air outside the isolator 10 to the working chamber 22, and includes a solenoid valve 40 and a fan 41.

The solenoid valve 40 supplies external air to the fan 41 under control of the control device 25. The fan 41 supplies the air supplied from the solenoid valve 40 to the working chamber 22.

### <Working Chamber>

The working chamber 22 is a space where work on cells and the like are conducted, and the working chamber 22 is provided with air filters 50 and 51, a door 52, the atomizer 100, and a working glove 53.

The air filter 50 is a filter for removing impurities such as dust contained in the air supplied from the fan 41. The air filter 51 is a filter for removing impurities such as dust contained in gas and the like which are discharged from the working chamber 22. Note that, for example, HEPA (High Efficiency Particulate Air) filters are used for the air filters 50 and 51.

The door 52 is provided in an openable/closable manner on the front face of the working chamber 22, so as to allow cells and the like to be brought into the working chamber 22.

The working glove 53 is attached to an opening (not shown) provided to the door 52 so that a worker can work on cells and the like in the working chamber 22 with the door 52 being closed. Note that the working chamber 22 is sealed when the door 52 is closed.

The atomizer 100 sprays hydrogen peroxide gas to decontaminate the interior of the working chamber 22.

### <Discharge Device>

The discharge device 23 is a device for discharging gas such as hydrogen peroxide gas, air, and the like from the working chamber 22, and includes a solenoid valve 60, a decontaminating gas inactivating device 61, and a fan 62.

The solenoid valve 60 supplies gas outputted from the air filter 51 to the decontaminating gas inactivating device 61 under control of the control device 25.

The decontaminating gas inactivating device 61 includes a catalyst, and renders harmless the gas outputted from the solenoid valve 60 for output to the fan 62.

The fan 62 outputs the gas outputted from the decontaminating gas inactivating device 61 to the exterior of the isolator 10 under control of the control device 25.

### <Operation Unit>

The operation unit 24 is an operation panel or the like for a user to set the operation of the isolator 10. The operation results of the operation unit 24 are transmitted to the control device 25, and the control device 25 controls each of the blocks of the isolator 10 based on the operation results.

### <Control Device>

The control device 25 is a device configured to perform an integrated control of the isolator 10, and includes a storage device 70 and a microcomputer 71.

The storage device 70 stores program data to be executed by the microcomputer 71 and various data. The microcomputer 71 implements various functions by executing the program data stored in the storage device 70. For example, when an instruction to generate decontaminating gas is outputted from the operation unit 24, the microcomputer 71 executes the predetermined program for generating decontaminating gas, and controls the air compressor 80, the pump 33, and the like.

A description will be given of functional blocks to be implemented by the microcomputer 71.

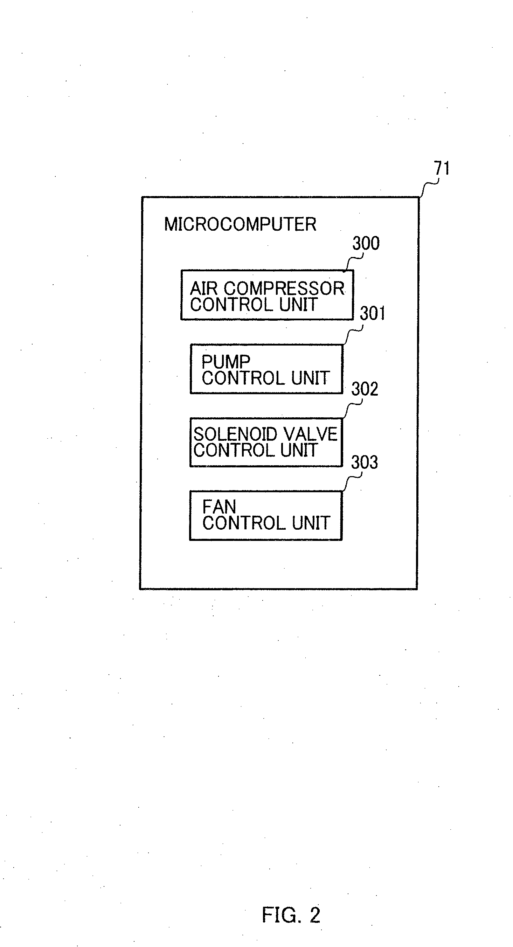

The microcomputer 71 executes the predetermined program stored in the storage device 70, and implements functions of an air compressor control unit 300, a pump control unit 301, a solenoid valve control unit 302, and a fan control unit 303 illustrated in Fig. 2.

### [Air Compressor Control Unit]

The air compressor control unit 300 starts the operation of the air compressor 80 when an instruction to generate decontaminating gas is outputted from the operation unit 24.

Further, the air compressor control unit 300 stops the operation of the air compressor 80 after, for example, a predetermined time has elapsed since the pump control unit 301 has stopped the operation of the pump 33. By performing the operation as such, it becomes possible to keep the pump 33 from pumping up the hydrogen peroxide solution while the air compressor 80 is not operating, thereby being able to improve the safety of the isolator 10. Note that, it is a matter of course that the air compressor control unit 300 may be configured to stop the operation of the air compressor 80 based on an instruction to stop the process sent from the operation unit 24.

### [Pump Control Unit]

The pump control unit 301 operates the pump 33 when the air compressor control unit 300 starts the operation of the air compressor 80. For example, the pump control unit 301 starts the operation of the pump 33 after a predetermined time has elapsed since the start of the operation of the air compressor 80 by the air compressor control unit 300. By performing an operation as such, it becomes possible to keep the pump 33 from pumping up the hydrogen peroxide solution while the air compressor 80 is not operating, thereby being able to improve safety of the isolator 10.

Further, the pump control unit 301 stops the pump 33 based on an instruction to stop the process sent from the operation unit 24. Note that the pump control unit 301 may be configured to stop the pump 33 when the air compressor control unit 300 stops the operation of the air compressor 80.

Further, the pump control unit 301 stops the pump 33 when receiving, from the water-level sensor 72, a signal indicating that a predetermined amount of the hydrogen peroxide solution has been stored in the bottle 31.

### [Solenoid Valve Control Unit]

The solenoid valve control unit 302 opens the solenoid valves 40 and 60, for example, when an instruction to ventilate the interior of the working chamber 22 is outputted from the operation unit 24. Further, the solenoid valve control unit 302 closes the solenoid valves 40 and 60, for example, when an instruction to stop the ventilation in the working chamber 22 is outputted from the operation unit 24. Note that the opening/ closing of the solenoid valves 40 and 60 may be controlled independently.

### [Fan Control Unit]

The fan control unit 303 starts the operations of the fans 41 and 62, for example, when an instruction to ventilate the working chamber 22 is outputted from the operation unit 24. Further, the fan control unit 303 stops the operations of the fans 41 and 62, for example, when an instruction to stop the ventilation in the working chamber 22 is outputted from the operation unit 24. Note that the operations of the fans 41 and 62 may be controlled independently.

### == Second Embodiment ==

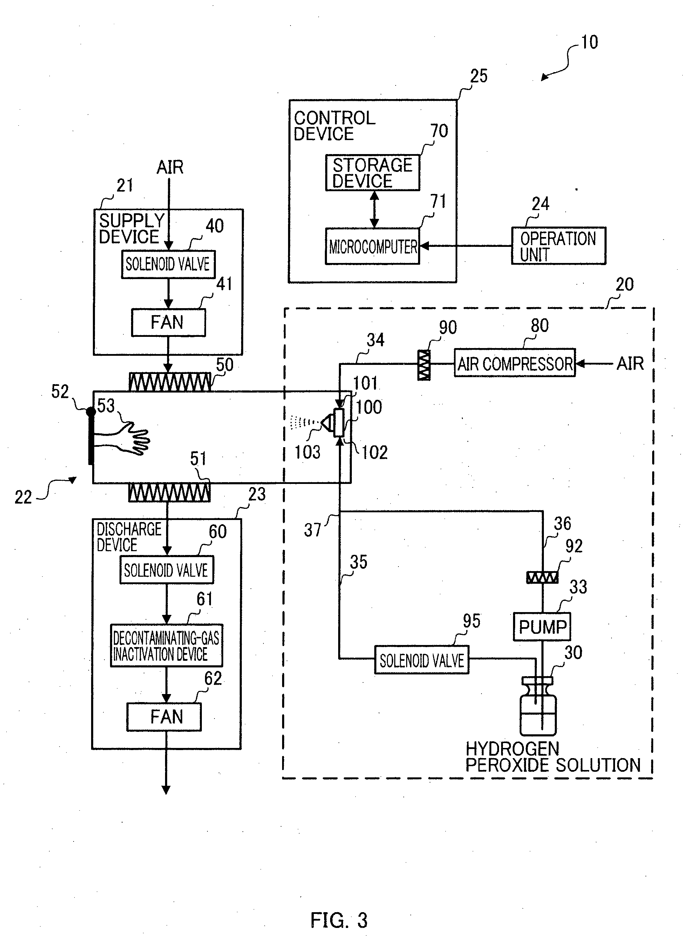

Fig. 3 is a diagram illustrating a configuration of an isolator 10 according to a second embodiment of the present invention. The isolator 10 is a device configured to conduct work on cells and the like under a decontaminated environment, and includes a decontamination solution spray device 20, a supply device 21, a working chamber 22, a discharge device 23, an operation unit 24, and a control device 25.

The isolator 10 according to a second embodiment of the present invention is different, as compared with the isolator of the first embodiment, in that the other end of the second pipe 35 of the decontamination solution spray device 20 is led into the interior of the tank 30 and that a solenoid valve 95 is provided between the other end of the second pipe 35 and the junction 37.

The solenoid valve control unit 302 of the microcomputer 71 opens the solenoid valve 95 when an instruction to generate decontaminating gas is outputted from the operation unit 24. Further, the solenoid valve control unit 302 closes the solenoid valve 95 when an instruction to stop the generation of decontaminating gas is outputted from the operation unit 24.

Also in the decontamination solution spray device 20 according to a second embodiment of the present invention, even if the pump 33 does not stop operating although air supply to the atomizer 100 is stopped due to some failure, for example, the hydrogen peroxide solution pumped up by the pump 33 does not rise up to the atomizer 100, and thus it becomes possible to prevent the hydrogen peroxide solution from being supplied to the atomizer 100. As a result, the hydrogen peroxide solution remaining in the form of liquid can be reliably prevented from being injected to the interior of the working chamber 22.

Further, the configuration of a second embodiment of the present invention can negate the need for the bottle 31, which receives the hydrogen peroxide solution running down from the other end of the second pipe 35.

Further, since the hydrogen peroxide solution which has not been sprayed flows back to the tank 30, the hydrogen peroxide solution can easily be reused. Further, the management of the bottle 31 becomes unnecessary, thereby being able to reduce the load of maintenance.

Further, even if the hydrogen peroxide solution which has not been sprayed from the atomizer 100 is returned to the tank 30, the amount of the hydrogen peroxide solution stored in the tank 30 would not exceed the initial amount and thus the hydrogen peroxide solution does not overflow from the tank 30. Therefore, the need for the water-level sensor 72 can be eliminated and safety of the isolator 10 can be improved.

Further, since the solenoid valve 95 is closed while the hydrogen peroxide solution is not being sprayed from the atomizer 100, impurities such as dust contained in the external air can be prevented from intruding into the working chamber 22 through the second pipe 35 and the atomizer 100.

Note that the other end of the second pipe 35 illustrated in Fig. 3 is provided in the interior of the tank 30, but may be provided vertically above the tank 30. In this case, the hydrogen peroxide solution returned to the tank 30 drips from the other end of the second pipe 35 into the tank 30.

### == Third Embodiment ==

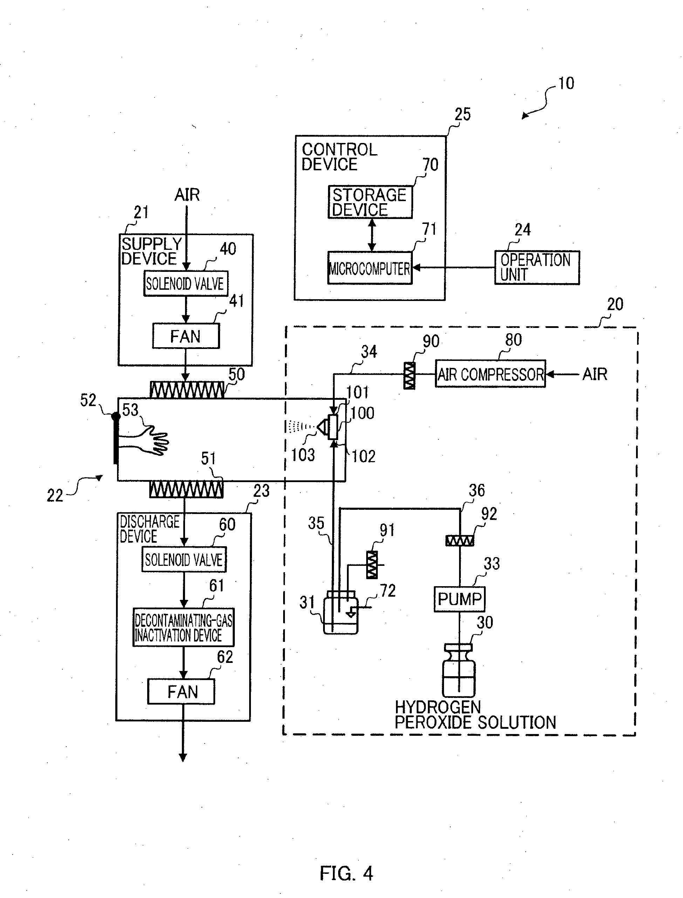

Fig. 4 is a diagram illustrating a configuration of an isolator 10 according to a third embodiment of the present invention. The isolator 10 is a device configured to conduct work on cells and the like under a decontaminated environment, and includes a decontamination solution spray device 20, a supply device 21, a working chamber 22, a discharge device 23, an operation unit 24, and a control device 25.

The isolator 10 according to a third embodiment of the present invention is different, as compared with the isolator of a first embodiment, in that the other end of the third pipe 36 is inserted into the interior of the bottle 31. In a third embodiment of the present invention, the hydrogen peroxide solution pumped up by the pump 33 from the tank 30 is injected from the other end of the third pipe 36 into the bottle 31.

In a third embodiment of the present invention, the other end of the second pipe 35 is provided so to be positioned in the hydrogen peroxide solution injected into the bottle 31. For example, the other end of the second pipe 35 is provided at a position in the vicinity of the inner bottom face of the bottle 31. In this case, even if the amount of the hydrogen peroxide solution to be stored in the bottle 31 is minute, the other end of the second pipe 35 can be set at a position in the hydrogen peroxide solution.

Further, the other end of the second pipe 35 can be controlled to remain at a position in the hydrogen peroxide solution by providing in the bottle 31 a sensor (not shown) which detects that the amount of the hydrogen peroxide solution in the bottle 31 is below the predetermined amount, and by driving the pump 33 in accordance with an instruction from the control device 25 to refill the hydrogen peroxide solution in the bottle 31 when the amount of the hydrogen peroxide solution in the bottle 31 falls below the predetermined amount.

Hereinabove, the decontamination solution spray device 20 according to first to third embodiments of the present invention have been described by way of example, and with such a decontamination solution spray device 20, the hydrogen peroxide solution can be prevented from being directly sprayed and dripping, even when air supply to the atomizer 100 is not carried out appropriately.

Further, the decontamination solution spray device 20 enables effective decontamination of the interior of the working chamber 22. For example, the hydrogen peroxide solution gas in the form of a mist can be effectively generated and sprayed in the working chamber 22, without using a vaporizer utilizing a heater or ultrasonic waves or the like when vaporizing the hydrogen peroxide solution.

Further, in the decontamination solution spray device 20, for example, even when air supply to the atomizer 100 is stopped due to some failure, the hydrogen peroxide solution remaining in the interior of the second pipe 35 does not rise up to the second port 102 of the atomizer 100, but drops from the other end of the second pipe 35 due to its own weight and is collected in the bottle 31 or the bottle 30. Further, the hydrogen peroxide solution sent out from the pump 33 is also collected in the bottle 31.

Therefore, the decontamination solution spray device 20 according to an embodiment of the present invention can prevent the hydrogen peroxide solution from being directly injected in the form of liquid to the interior of the working chamber 22. As a result, a worker can safely conduct work such as cell culture and the like in the working chamber 22.

For this reason, the atomizer 100 can be provided in the interior of the working chamber 22, and thus decontamination in the interior of the working chamber 22 can be effectively performed. For example, as compared with the case of the atomizer 100 provided in the exterior of the working chamber 22, hydrogen peroxide with a higher concentration can be sprayed from the atomizer 100, thereby being able to achieve an increased decontamination effect.

For example, in the embodiments of the present invention, hydrogen peroxide solution has been given by way of example as a decontamination solution, however, alcohols such as ethanol and isopropyl alcohol, hypochlorous acid solution, chlorine dioxide solution, ozone water, formaldehyde and the like may be used.

### [Reference Signs List]

- 10: isolator
- 20: decontamination solution spray device
- 21: supply device
- 22: working chamber
- 23: discharge device
- 24: operation unit
- 25: control device
- 30: tank
- 31: bottle
- 33: pump
- 34: first pipe
- 35: second pipe
- 36: third pipe
- 37: junction
- 40,60,95: solenoid valve
- 41: fan
- 50, 51: air filter
- 52: door
- 53: glove
- 61: decontaminating gas inactivating device
- 62: fan
- 70: storage device
- 71: microcomputer
- 72: water-level sensor
- 80: air compressor
- 90,91: air filter
- 92: filter
- 100: spray
- 101: first port
- 102: second port
- 103: nozzle
- 300: air compressor control unit
- 301: pump control unit
- 302: solenoid valve control unit
- 303: fan control unit

## Claims

1. A decontamination solution spray device comprising:
an atomizer (100) including a first port (101), a second port (102), and a nozzle (103);
a first pipe (34) having one end connected to an air compressor (80) and an other end connected to the first port (101) ;
a second pipe (35) provided lower than the second port (102), the second pipe (35) having one end connected to the second port (102) and an other end open;
a reservoir portion (30) configured to store a decontamination solution;
a pump (33) configured to pump up the decontamination solution from the reservoir portion (30); and
a third pipe (36), having one end connected to the pump (33), through which the decontamination solution taken in by the pump (33) flows,
the atomizer (100) configured to, suck the decontamination solution flowing through the third pipe (36) via the second pipe (35), by negative pressure produced in the second port (102) by injecting air taken in from the first port (101) from the nozzle (103); and inject the decontamination solution in an atomized state from the nozzle (103), mixing the decontamination solution with air,
**characterised in that**
an other end of the third pipe (36) joins the second pipe (35) at a junction on a path of the second pipe.

2. The decontamination solution spray device according to claim 1, further comprising
a bottle (31) provided vertically below the other end of the second pipe (35), the bottle (31) configured to receive the decontamination solution running down from the junction (37).

3. The decontamination solution spray device according to claim 2, further comprising:
a sensor (72) configured to detect that the decontamination solution which has run down from the second pipe (35) has been stored in the bottle (31); and
a pump control unit (301) configured to stop the pump (33) when a signal indicative of an effect that the decontamination solution has been stored in the bottle (31) is received from the sensor.

4. The decontamination solution spray device according to claim 1, wherein
the other end of the second pipe (35) is provided vertically above the reservoir portion (30) or in an interior thereof.

5. The decontamination solution spray device according to claim 1, further comprising
a bottle (31) configured to store the decontamination solution pumped up by the pump (33), wherein
an other end of the third pipe (36) is provided in an interior of the bottle (31), and
the other end of the second pipe (35) is provided such that the other end is positioned in the decontamination solution stored in the bottle (31).

6. The decontamination solution spray device according to any one of claims 1 to 5,
wherein
the decontamination solution includes hydrogen peroxide solution.

7. The decontamination solution spray device according to any one of claims 1 to 6,
wherein
the atomizer (100) is provided in a working chamber (22) where work on a cell is to be conducted.

## Patentansprüche

1. Vorrichtung zum Versprühen von Dekontaminations-Lösung, die umfasst:
einen Zerstäuber (100), der einen ersten Anschluss (101), einen zweiten Anschluss (102) und eine Düse (103) enthält;
ein erstes Rohr (34), dessen eines Ende mit einem Drucklufterzeuger (80) verbunden ist und dessen anderes Ende mit dem ersten Anschluss (101) verbunden ist;
ein zweites Rohr (35), das tiefer angeordnet ist als der zweite Anschluss (102), wobei ein Ende des zweiten Rohrs (35) mit dem zweiten Anschluss (102) verbunden ist und das andere Ende offen ist;
einen Behälterabschnitt (30), der zum Speichern einer Dekontaminations-Lösung eingerichtet ist;
eine Pumpe (33), die zum Hochpumpen der Dekontaminations-Lösung aus dem Behälterabschnitt (30) eingerichtet ist; und
ein drittes Rohr (36), dessen eines Ende mit der Pumpe (33) verbunden ist und durch das die durch die Pumpe (33) eingeleitete Dekontaminations-Lösung fließt,
wobei der Zerstäuber (100) so eingerichtet ist, dass er die durch das dritte Rohr (33) fließende Dekontaminations-Lösung über das zweite Rohr mittels Unterdruck ansaugt, der an dem zweiten Anschluss (102) erzeugt wird, indem über den ersten Anschluss (101) eingeleitete Luft über die Düse (103) eingeblasen wird, und dass er die Dekontaminations-Lösung in einem zerstäubten Zustand über die Düse (103) verspritzt und die Dekontaminations-Lösung mit Luft vermischt,
**dadurch gekennzeichnet, dass**
sich ein anderes Ende des dritten Rohrs (36) an einer Verbindungsstelle auf einem Weg des zweiten Rohrs an das zweite Rohr (35) anschließt.

2. Vorrichtung zum Versprühen von Dekontaminations-Lösung nach Anspruch 1, die des Weiteren umfasst:
eine Flasche (31), die sich vertikal unterhalb des anderen Endes des zweiten Rohrs (35) befindet, wobei die Flasche (31) so eingerichtet ist, dass sie die Dekontaminations-Lösung aufnimmt, die über die Verbindungsstelle (37) nach unten läuft.

3. Vorrichtung zum Versprühen von Dekontaminations-Lösung nach Anspruch 2, die des Weiteren umfasst:
einen Sensor (72), der so eingerichtet ist, dass er erfasst, dass die Dekontaminations-Lösung, die über das zweite Rohr (35) nach unten gelaufen ist, in der Flasche (31) gespeichert worden ist; und
eine Pumpen-Steuereinheit (301), die so eingerichtet ist, dass sie die Pumpe (33) anhält, wenn von dem Sensor ein Signal empfangen wird, das einen Effekt dahingehend anzeigt, dass die Dekontaminations-Lösung in der Flasche (31) gespeichert worden ist.

4. Vorrichtung zum Versprühen von Dekontaminations-Lösung nach Anspruch 1, wobei sich das andere Ende des zweiten Rohrs (35) vertikal oberhalb des Behälterabschnitts (30) oder in seinem Inneren befindet.

5. Vorrichtung zum Versprühen von Dekontaminations-Lösung nach Anspruch 1, die des Weiteren umfasst:
eine Flasche (31), die so eingerichtet ist, dass sie die durch die Pumpe (33) hochgepumpte Dekontaminations-Lösung speichert, wobei
ein anderes Ende des dritten Rohrs (36) in einem Inneren der Flasche (31) angeordnet ist, und
das andere Ende des zweiten Rohrs (35) so angeordnet ist, dass sich das andere Ende in der in der Flasche (31) gespeicherten Dekontaminations-Lösung befindet.

6. Vorrichtung zum Versprühen von Dekontaminations-Lösung nach einem der Ansprüche 1 bis 5, wobei
die Dekontaminations-Lösung Wasserstoffperoxid-Lösung enthält.

7. Vorrichtung zum Versprühen von Dekontaminations-Lösung nach einem der Ansprüche 1 bis 6, wobei
sich der Zerstäuber (100) in einer Arbeitskammer (22) befindet, in der Arbeit an einer Zelle ausgeführt wird.

## Revendications

1. Dispositif de pulvérisation de solution de décontamination comprenant :
un atomiseur (100) incluant un premier orifice (101), un second orifice (102) et une buse (103) ;
un premier tuyau (34) ayant une extrémité raccordée à un compresseur d'air (80) et une autre extrémité raccordée au premier orifice (101) ;
un deuxième tuyau (35) disposé plus bas que le second orifice (102), le deuxième tuyau (35) ayant une extrémité raccordée au second orifice (102) et une autre extrémité ouverte ;
une partie formant réservoir (30) configurée pour stocker une solution de décontamination ;
une pompe (33) configurée pour pomper la solution de décontamination depuis la partie formant réservoir (30) ; et
un troisième tuyau (36) ayant une extrémité raccordée à la pompe (33) à travers laquelle s'écoule la solution de décontamination aspirée par la pompe (33),
l'atomiseur (100) étant configuré pour aspirer la solution de décontamination circulant à travers le troisième tuyau (36) par l'intermédiaire du deuxième tuyau (35) au moyen d'une pression négative produite dans le second orifice (102) en injectant de l'air aspiré depuis le premier orifice (101) par la buse (103) ; et
injecter la solution de décontamination dans un état atomisé depuis la buse (103), mélanger la solution de décontamination avec de l'air, **caractérisé en ce que**
une autre extrémité du troisième tuyau (36) raccorde le deuxième tuyau (35) au niveau d'une jonction sur le trajet du deuxième tuyau.

2. Dispositif de pulvérisation de solution de décontamination selon la revendication 1, comprenant en outre
une bouteille (31) disposée verticalement au-dessous de l'autre extrémité du deuxième tuyau (35), la bouteille (31) étant configurée pour recevoir la solution de décontamination descendant de la jonction (37).

3. Dispositif de pulvérisation de solution de décontamination selon la revendication 2, comprenant en outre :
un détecteur (72) configuré pour détecter que la solution de décontamination passant dans le deuxième tuyau (35) a été stockée dans la bouteille (31) ; et
une unité de commande de pompe (301) configurée pour arrêter la pompe (33) lorsqu'un signal indiquant un effet tel que la solution de décontamination a été stockée dans la bouteille (31) et reçu depuis le détecteur.

4. Dispositif de pulvérisation de solution de décontamination selon la revendication 1, dans lequel
l'autre extrémité du deuxième tuyau (35) est disposée verticalement au-dessus de la partie formant réservoir (30) ou à l'intérieur de celle-ci.

5. Dispositif de pulvérisation de solution de décontamination selon la revendication 1, comprenant en outre
une bouteille (31) configurée pour stocker la solution de décontamination pompée par la pompe (33), dans lequel
une autre extrémité du troisième tuyau (36) est prévue à l'intérieur de la bouteille (31), et
l'autre extrémité du deuxième tuyau (35) est prévue de sorte que l'autre extrémité est positionnée dans la solution de décontamination stockée dans la bouteille (31).

6. Dispositif de pulvérisation de solution de décontamination selon l'une quelconque des revendications 1 à 5, dans lequel
la solution de décontamination contient une solution de peroxyde d'hydrogène.

7. Dispositif de pulvérisation de solution de décontamination selon l'une quelconque des revendications 1 à 6, dans lequel
l'atomiseur (100) est prévu dans une chambre de travail (22) où le travail sur une cellule doit être effectué.
